(19) Europäisches Patentamt
European Patent Office
Office européen des brevets

(11) Numéro de publication: 0 188 941
B1

(12) FASCICULE DE BREVET EUROPEEN

(45) Date de publication du fascicule du brevet: 12.04.89

(51) Int. Cl.⁴: C 09 B 47/04, C 09 K 19/40, C 07 D 487/22 // (C07D487/22, 259:00, 209:00, 209:00, 209:00, 209:00)

(21) Numéro de dépôt: 85402498.1

(22) Date de dépôt: 16.12.85

# (54) Cristaux liquides electrochromes à base de phtalocyanine substituée.

(30) Priorité: 18.12.84 FR 8419348

(43) Date de publication de la demande: 30.07.86 Bulletin 86/31

(45) Mention de la délivrance du brevet: 12.04.89 Bulletin 89/15

(84) Etats contractants désignés: CH DE GB IT LI NL

(73) Titulaire: ETAT FRANCAIS représenté par le Ministre des PTT (Centre National d'Etudes des Télécommunications), 38-40 rue du Général Leclerc, F-92131 Issy-les-Moulineaux (FR)
Titulaire: ESPCI Ecole Supérieure de Physique et de Chimie Industrielles de la Ville de Paris, 10, rue Vauquelin, F-75231 Paris Cédex 05 (FR)

(72) Inventeur: Clarisse, Christian, 13 Résidence de Pors Ty Olu, Saint Quay, Perros, F-22700 Perros-Guirec (FR)
Inventeur: Riou née Meston, Marie-Thérèse, 8, rue Etienne d'Orves, F-22700 Perros-Guirec (FR)
Inventeur: Simon, Jacques, 9 Villa Saint Mandé, F-75012 Paris (FR)
Inventeur: Piechocki, Christian, 9, rue Saint Wendelin, F-67501 Haguenau (FR)

(74) Mandataire: Warcoin, Jacques et al, Cabinet Régimbeau 26, avenue Kléber, F-75116 Paris (FR)

(56) Documents cités:
EP-A- 0 134 518
EP-A- 0 140 133
CH-A- 414 044
FR-A- 1 207 348
FR-A- 1 389 808
FR-A- 1 580 683
GB-A- 2 058 114

MOLECULAR CRYSTALS AND LIQUID CRYSTALS + LETTERS, vol. 100, no. 3/4, 1983, pages 275-284, Gordon and Breach, Science Publishers, Inc., New York, US; D. GUILLON et al.: "Discotic mesophases of the metal-free derivative of octa (dodecyloxymethyl) phthalocyanine"

CHEMICAL ABSTRACTS, vol. 100, no. 10, mars 1984, page 79, abrégé no. 69847g, Columbus, Ohio, US; L.G. TOMILOVA et al.: "Phthalocynines and related compounds. XXII. Synthesis and spectroscopic-electrochemical study of rare earth element diphthalocyanines" & ZH. OBSHCH. KHIM. 1983, 53(11), 2594-601

C. Piechocki et al. J. Am. Chem. Soc. 104, 5245 (1982)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 188 941 B1

ACTORUM AG

## Description

La présente invention concerne de nouveaux composés organométalliques, des procédés pour leur préparation ainsi que l'application de ces composés dans le domaine de l'électronique et de l'optoélectronique.

Les nouveaux composés organométalliques selon la présente invention sont constitués de deux parties. Tout d'abord un ligand capable de complexer un ion métallique; plus particulièrement il s'agit de composés dans lesquels le ligand est une phtalocyanine ou une porphyrine et le métal peut être de nature variée.

Le ligand constitué par une porphyrine ou une phtalocyanine présente des substituants de type paraffinique dont la nature ainsi que celle du métal permettent d'obtenir des phases organisées de différents types et de propriétés variées.

En particulier, les composés selon la présente invention sont susceptibles de constituer des mésophases de type discotique, ceci correspond à une organisation dans laquelle le ligand aromatique central est entouré par plusieurs chaînes paraffiniques flexibles, les cœurs rigides de ces composés constitués du cycle phtalocyanine ou porphyrine viennent s'empiler les uns sur les autres pour former des colonnes qui cristallisent sous forme hexagonale bidimensionnelle.

Les composés selon la présente invention sont donc susceptibles de conduire à la formation de cristaux liquides discotiques; en outre, la présence d'un ion métallique dans la structure permet de leur conférer des propriétés électrochromes, c'est-à-dire que la couleur des composés varie suivant les caractéristiques du potentiel électrique qui leur est appliqué.

L'existence de phases discotiques a déjà été rapportée pour différents composés et l'existence de propriétés électrochromes pour des dérivés de phtalocyanine, comme par exemple la phtalocyanine de lutécium, a été également rapportée; mais, à la connaissance des Demandeurs, il n'a jamais été rapporté l'existence de cristaux liquides, en particulier discotiques, présentant des propriétés électrochromes.

De nombreuses publications concernent des dérivés de phtalocyanine. Ainsi, le brevet EP-A-0 134 518, qui est opposable à la présente demande au sens de l'article 54(3) et (4) de la CBE, concerne des dérivés de naphtalocyanine. On peut également citer les articles suivants: C. Piechocki et al. J.Am. Chem. Soc. 104, 5245 (1982), D. Guillon et al. Mol. Cryst. Liq. Cryst. 100, 275 (1983), Chem. Abstr. 100 (1984), 69847 g.

Ce type de composant peut présenter un intérêt très important en électronique et en opto-électronique, en particulier dans les dispositifs d'affichage où on utilise déjà des dérivés de diphtalocyanines de terres rares.

Il est inutile de rappeler ici toutes les publications ayant trait à l'utilisation de diphtalocyanines de terres rares dans les dispositifs d'affichage, pas plus que l'utilisation des cristaux liquides dans ce même type de dispositif.

On a déjà tenté d'utiliser des systèmes basés sur un cristal liquide présentant des propriétés électrochromes en utilisant des cristaux liquides usuels tels que le 4-n-octyl-4'-cyanobiphényle dans lequel on a ajouté un électrolyte, en l'occurrence l'iodure de tétra-n-butylammonium. Ce type de mélange montre un changement de couleur de l'incolore vers le bleu-vert lorsqu'on applique un champ électrique, K. Nakamura, S. Kanebo, Y. Ito, H. Hirabayashi, K. Ogura, J. Appl. Phys. 53, 1792 (1982).

Le mécanisme mis en œuvre est encore pour l'instant largement incompris.

On a, de même, proposé d'autres mélanges de cristaux liquides et d'un colorant ou d'une molécule fluorescente afin de combiner les propriétés des cristaux liquides et des composés électrochromes, A. Hochbaum, L.J. Yu, M.M. Labes, J. Appl. Phys. 51, 867 (1980).

Mais il n'existe pour l'instant aucun composé présentant en lui-même les deux propriétés, à savoir: des propriétés électrochromes à l'état cristallin liquide.

Les composés selon la présente invention sont des composés organométalliques de formule:

$$[(R')_n (R'')_m \text{Por}]_p \text{Me} \qquad (I)$$

dans laquelle:

– Por représente un radical choisi parmi les radicaux:
  – phtalocyanine,
  – porphyrine,
  – tétraazaporphyrine,
  – tétra(naphto)tétraazaporphyrine,
  – tétra(phénanthro)tétraazaporphyrine,
– R' et R'' représentent des radicaux identiques ou différents, fixés sur les atomes de carbone périphériques du cycle Por;
– R' est un radical à caractère aliphatique dont la chaîne carbonée peut être interrompue par un ou plusieurs hétéroatomes choisis parmi O, S, N et Se ou par un radical phénylène, la chaîne carbonée contenant au moins 6 atomes;
– R'' est un radical cyano ou halogéno;
– n et m sont des nombres entiers dont la somme est au moins égale à 4 et au plus égale au nombre d'atomes de carbone périphériques du cycle Por;
– n au moins égal à 4,
– p est égal à 1 ou 2;
– Me est un métal.

avec la précision que quand le cposé n'est pas dissymétrique p est égal à 1, Me est choisi dans le groupe constitué par le groupe des lanthanides et de l'yttrium, l'uranium, le thorium, l'étain, le platine, le palladium, le manganèse et le plomb; et lorsque Por est une tétra(naphto)tétraazaporphyrine, n est supérieure à 4.

Dans la formule précédente, on remarquera que les composés de formule I doivent présenter de préférence plus de 4 chaînes aliphatiques latérales, ceci afin de permettre d'obtenir une phase organisée, en particulier une phase discotique. De même, afin que les phases organisées puissent apparaître, il est important que la chaîne carbonée soit suffisamment longue, c'est-à-dire qu'elle contienne au moins 6 atomes, que ceux-ci soient des atomes de carbone ou des hétéroatomes.

Par «atomes de carbone périphériques» on entend désigner les atomes de carbone les plus éloignés du centre géométrique du cycle Por.

De même, pour la valeur de p, cette valeur dépendra de la nature du métal utilisé et, éventuellement, de son degré d'oxydation.

Parmi les composés selon la présente invention, on préfère tout particulièrement les composés organométalliques dans lesquels le cycle central est le cycle de la phtalocyanine, c'est-à-dire des composés de formule:

$$[(R)_nPc]_p Me$$

dans laquelle:

– Pc symbolise le radical phtalocyanine substitué suivant:

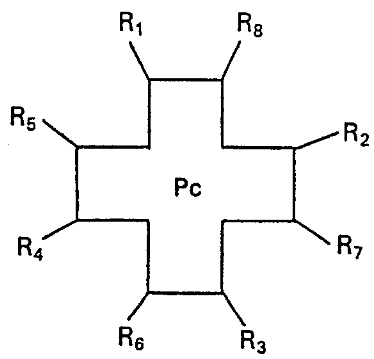

(I')

– $R_1$ à $R_8$ représentent H ou l'une des valeurs données pour R' et R'', sous réserve qu'au moins 4 des radicaux $R_1$ à $R_8$ soient des radicaux aliphatiques.

Le radical phtalocyanine a été simplifié et représenté uniquement par les atomes de carbone périphériques, sa structure complète est représentée à la figure 1.

De façon encore préférable, dans ces composés les radicaux $R_1$ à $R_8$ représentent H ou un radical choisi parmi les radicaux alcoyles en $C_6$ à $C_{50}$, droits ou ramifiés, les radicaux alcoyles en $C_6$ à $C_{50}$ comportant un ou plusieurs hétéroatomes choisis parmi O, S ou N dans la chaîne.

Pour des raisons tenant à la simplicité de la synthèse et également à la symétrie de la molécule, les composés selon la présente invention seront de préférence ceux dans lesquels tous les substituants $R_1$ à $R_8$ seront identiques et $R_1$ à $R_8$ représenteront les radicaux $-CH_2-O-R_{10}$, $R_{10}$ étant un radical alcoyle en $C_4$ à $C_{50}$ ou une chaîne alcoylée interrompue par un ou plusieurs hétéroatomes choisis parmi O, S ou N; et de façon encore préférée $R_{10}$ sera choisi parmi:

$-C_8H_{17}$; $-C_{12}H_{25}$; $-CH(CH_2-O-C_{12}H_{25})_2$;

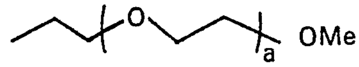

a = 1,7 ou 14

Lorsque la longueur des chaînes devient trop importante, ceci peut gêner l'organisation des cycles et les cristaux, lorsqu'ils sont obtenus, peuvent avoir une organisation non-discotique.

Parmi les composés préférés selon la présente invention, il faut citer les composés de formules Ia, Ib, Ic, Id, Ie et If dans lesquels le radical Pc représente les formules 1a, 1b, 1c, 1d, 1e et 1f qui sont schématisés à la figure 1.

Les métaux mis en œuvre peuvent être très variés; il s'agit de préférence de métaux du groupe des lanthanides et de l'yttrium tels que le lutécium ou le dysprosium par exemple, ou bien des métaux tels que l'uranium, le thorium ou l'étain.

Les métaux du groupe des lanthanides donnent des composés organométalliques dans lesquels chaque atome métallique est pris en «sandwich» entre deux cycles phtalocyanine. Mais, il est possible de réaliser des composés avec un seul cycle par atome métallique avec des métaux tels que le plomb par exemple.

Les composés particulièrement intéressants pour leurs propriétés de cristaux liquides et leurs propriétés électrochromes sont les composés Ia et If qui seront étudiés plus en détail dans les exemples.

Dans certains cas les composés synthétisés peuvent être dissymétriques, dans ce cas on préfèrera les composés dans lesquels $R_1$ et $R_8$ représentent le radical cyano, ces composés présentent la particularité de présenter un moment dipolaire dans le plan très important qui peut les rendre précieux dans les dispositifs électroniques ou opto-électroniques.

Dans d'autres cas, lorsque l'atome métallique a un rayon atomique suffisamment important, cet atome métallique ne peut se loger dans le plan de la porphyrine ou des phtalocyanines, il se trouve donc au-dessus du plan de la molécule et induit ainsi un moment dipolaire pour cette molécule qui sera perpendiculaire au plan, c'est le cas par exemple des composés organométalliques avec le plomb. Là encore, l'existence d'un moment dipolaire perpendiculaire à la molécule peut permettre des utilisations intéressantes de ces composés.

Les composés selon la présente invention peuvent être préparés par divers procédés En particulier, les composés organométalliques peuvent être préparés à partir du composé non métallé par action d'un sel du métal sur le dianion du ligand organique, de préférence le dianion est préparé par action d'un alcoolate de métal alcalin sur le composé organique.

Parmi les alcoolates utilisables, il faut citer des composés tels que l'amylate de potassium en solution dans l'alcool amylique.

La partie organique non métallée des composés selon l'invention peut être préparée notamment selon le schéma suivant:

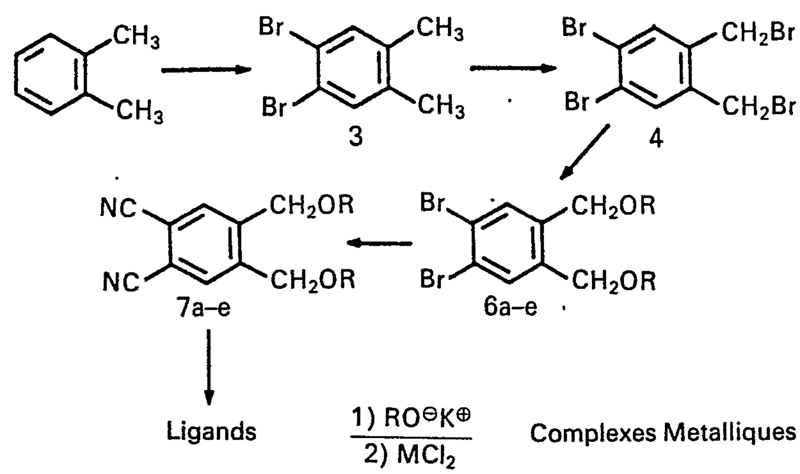


3, 4, 6a–e, 7a–e

Schéma 1

Dans ce procédé, l'oxylène est traité par le brome pour donner le dérivé 4,5-dibromé. Les groupements méthyles sont alors bromés avec le N-bromosuccinimide par un mécanisme radicalaire, ce qui conduit à des rendements supérieurs à la bromation directe par $Br_2$.

Les chaînes latérales paraffiniques sont alors greffées par réaction des alcoolates ROH correspondants avec le dérivé tétrabromé.

Cette réaction nécessite l'utilisation d'un solvant protique anhydre, par exemple un excès de l'alcool correspondant ou le t-butanol, de façon à éviter les réactions d'élimination qui surviennent dans les solvants aprotiques tels que le DMF ou le THF.

Le dérivé dicyané correspondant (7) est obtenu en traitant le dérivé dibromé (6) par le cyanure cuivreux dans le DMF. La concentration des réactifs doit être suffisamment faible pour éviter la formation de la phtalocyanine de cuivre. En partant du dérivé dicyané (7) on peut préparer la phtalocyanine correspondante par diverses routes.

Le composé (7) traité avec le lithium dans le 1-pentanol conduit au sel de dilithium de Pc.

Le composé (7) peut être également traité dans un solvant à haut point de fusion (1-chloronaphtalène ou quinoline par exemple) en présence de métal finement divisé ou d'un sel métallique pour donner la phtalocyanine métallée correspondante.

Pc peut également être obtenu électrochimiquement.

Dans le cas des dérivés octasubstitués, les meilleurs rendements sont obtenus en transformant le composé (7) en les 1,3-diimino-isoindolines correspondantes par traitement à l'ammoniac en milieu basique. Ces diimino-isoindoles sont dissous dans le 2-diméthylaminoéthanol et chauffés au reflux pour donner le dérivé correspondant non métallé (1).

Lorsque le produit à préparer doit comporter des radicaux cyano en position α, on part du dérivé tétracyané qui est traité par l'ammoniac et l'amylate de sodium, on obtient le composé (8) sur le schéma ci-après:

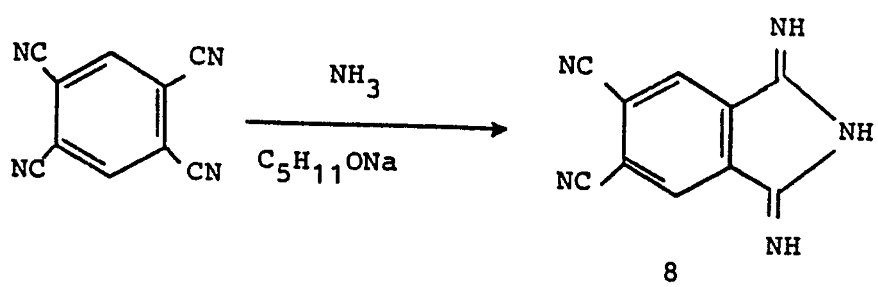


8

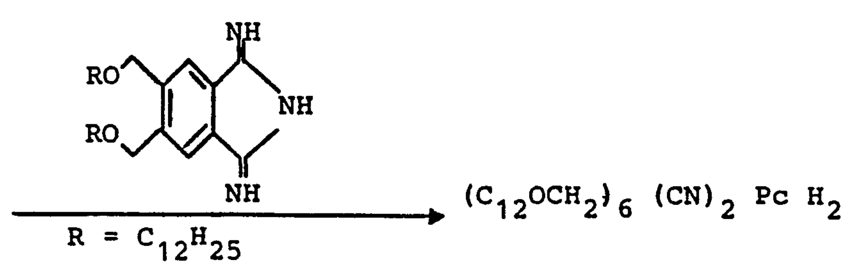


Schéma 2

La condensation dans un rapport de l'ordre de 1/3 du composé précédent et du composé correspondant non cyané conduit au dérivé de formule souhaitée.

Les composés selon la présente invention sont

utilisables en électronique et en opto-électronique, en particulier dans les dispositifs d'affichage comme cela est déjà connu pour les composés électrochromes ou les cristaux liquides mais en permettant d'utiliser au mieux les avantages des deux types de composant.

Sur les figures ci-annexées:

– la figure 1 représente la structure de certains composés selon l'invention non métallés,

– la figure 2 représente une vue en perspective de la structure du composé de l'exemple 16,

– la figure 3 représente une vue en perspective de la structure du composé de l'exemple 18,

– les figures 4 à 6 représentent des courbes d'analyse thermique différentielle pour le composé de l'exemple 18,

– la figure 7 représente un spectre RPE en fonction de la température pour le composé de l'exemple 18.

D'autres caractéristiques et avantages de la présente invention apparaîtront à la lecture des exemples ci-après.

Exemple 1 – Dibromo-4,5-o-xylène

220 g (2,06 moles) de o-xylène sont refroidis jusqu'à 5–10 °C, quelques mg de poudre de fer et un cristal d'iode sont ajoutés. Le catalyseur de bromation se trouve formé lorsque la coloration caractéristique de l'iode disparaît. 660 g de brome (4,1 moles) sont alors ajoutés goutte à goutte; on note le dégagement d'une importante quantité d'acide bromhydrique. Lorsqu'environ la moitié du brome total a été ajoutée, on rajoute 200 ml de chlorure de méthylène pour diluer le milieu réactionnel. Le restant du brome est ajouté et le mélange réactionnel est maintenu à 5–10 °C pendant environ 48 heures. 200 ml de chlorure de méthylène sont ajoutés et le brome en excès est neutralisé en traitant la phase organique avec une solution diluée de bisulfite de sodium. Les extraits organiques sont rincés à l'eau distillée, séchés sur $MgSO_4$ et évaporés jusqu'à siccité. La recristallisation à partir d'hexane donne des cristaux blancs.

Rendement: 40%; Tf: 86 °C; $^1H$ RMN ($CCl_4$): 2,1 (s, 6H, ∅$CH_3$), 7,15 (s, 2H, arom.).

| | | | |
|---|---|---|---|
| Analyse: | $C_8H_8Br_2$ (263,96): | | |
| Calculé (%): | C 36,40; | H 3,05; | Br 60,54; |
| Trouvé (%): | C 36,50; | H 3,11; | Br 60,65; |
| total: | 100,26%. | | |

Exemple 2 – 1,2-dibromo-4,5-bis(bromomethyl)-benzène

100 g (0,379 mole) du dérivé dibromé obtenu à l'exemple 1 sont mélangés avec 140 g (0,786 mole) de N-bromosuccinimide et 1,26 g (5,2 mmoles) de peroxyde de benzoyle dans 400 ml de tétrachlorure de carbone et chauffés sous reflux; la réaction radicalaire démarre approximativement 15 à 30 minutes après le début du chauffage à reflux. La réaction se continue jusqu'à ce que l'ébullition diminue. La solution chaude est filtrée et le filtrat est évaporé jusqu'à siccité. Le résidu huileux jaune est dissous dans l'éther de pétrole bouillant; la solution chaude est alors filtrée pour éliminer la succinimide qui a précipité. Le dérivé du titre est cristallisé à 5 °C.

Rendement: 60%; TF: 88 °C; $^1H$ RMN ($CCl_4$): 4,35 (s, 4H, ∅$CH_2Br$), 7,35 (s, 2H, arom.).

| | | | |
|---|---|---|---|
| Analyse: | $C_8H_6Br_4$ (421,75): | | |
| Calculé (%): | C 22,78; | H 1,44; | Br 75,78; |
| Trouvé (%): | C 22,83; | H 1,14; | Br 76,78; |
| total: | 100,75%. | | |

Exemple 3 – Hydroxy-15-nonacosaneoxy-dioxa-13,17

Le composé du titre est préparé en ajoutant goutte à goutte sous une atmosphère d'azote 23 g (0,249 mole) d'épichlorhydrine dans 300 g (1,61 mole) de 1-dodécanol contenant 20 g (0,5 mole) d'hydroxyde de sodium finement divisé. Le mélange est chauffé à 120 °C et agité pendant une nuit à cette température. A la température normale, 200 ml d'eau sont ajoutés. La phase aqueuse est alors extraite avec trois fois 150 ml de $CHCl_3$. Les extraits organiques combinés sont séchés sur $Na_2SO_4$, filtrés et évaporés jusqu'à siccité. Le résidu huileux est distillé sous vide (1,33 Pa ($10^{-2}$ Torr) ); le dodécanol est tout d'abord récupéré à environ 90 °C. Le dérivé du titre distille à une température comprise entre 180 et 210 °C. L'alcool ainsi obtenu est de nouveau purifié par recristallisation dans l'heptane.

Rendement: 47%; TF: 40 °C; $^1H$ RMN ($CDCl_3$): 1,2 (m, 46H, $-CH_2-$), 3,3 (m, 9H, $-CH_2O-$ et OH), 3,7 (m, 1H, $-\underline{CH}-OH$).

| | | | |
|---|---|---|---|
| Analyse: | $C_{27}H_{56}O_3$ (428,75): | | |
| Calculé (%): | C 75,64; | H 13,17; | O 11,19; |
| Trouvé (%): | C 75,57; | H 13,21; | O 11,59; |
| total: | 100,37%. | | |

Exemple 4 – 1,2-dibromo-4,5-bis-(dodécaneoxyméthyl)-benzène

On fait réagir 45 ml (0,47 mole) de t-butanol fraîchement distillé avec 4,63 g (0,118 mole) de potassium sous atmosphère d'azote. Après complète réaction du métal, 20 g (0,11 mole) de 1-dodécanol sont ajoutés en une seule fois à 40 °C. 10 g (0,024 mole) du dérivé préparé à l'exemple 2, en poudre, sont alors ajoutés par fraction, l'addition complète prenant environ 4 heures. Le mélange réactionnel est alors chauffé à 60 °C pendant 48 heures. On utilise la CCM pour mettre en évidence la fin de la réaction ($SiO_2$; éluent: heptane/toluène (1/1). $R_F$: 0,76 pour le composé du titre et $R_F$: 0,30 pour la matière de départ.

A la température ambiante, 200 ml d'eau distillée sont ajoutés. La phase aqueuse est neutralisée avec une solution diluée de HCl et extraite avec $CHCl_3$. Les extraits organiques combinés sont séchés sur $Na_2SO_4$, filtrés et évaporés jusqu'à siccité. L'excès de 1-dodécanol est distillé sous vide (75–100 °C; $6,65 \cdot 10^{-1}$ Pa ($5 \cdot 10^{-3}$ Torr). Le résidu huileux rougeâtre restant est filtré sur une colonne d'alumine (éluent: toluène) et extrait avec de l'éthanol bouillant (3 fois 200 ml). Le dérivé du titre est cristallisé à 5–7 °C.

Rendement: 47%; TF: 41–43 °C; $^1H$ RMN ($CCl_4$): 1,2 (m, 46H, $-CH_2-$), 3,3 (m, 4H, $-OCH_2-$), 4,25

(s, 4H, $\varnothing CH_2O$–), 7,4 (s, 2H, arom.). $^{13}C$ RMN ($CDCl_3$): 14,1, 22,6, 26,1, 29,5, 29,4, 29,3, 31,8, 69,1, 70,9, 123,4, 133,1, 137,4.

Analyse: $C_{32}H_{56}O_2Br_2$ (632,62):
Calculé (%): C 60,76; H 8,92; O 5,06; Br 25,25;
Trouvé (%): C 60,66; H 8,79; O 5,02; Br 25,63;
total: 100,1%.

Exemple 5 – Préparation du dérivé 6b

En opérant comme dans l'exemple 4, mais en remplaçant le 1-dodécanol par le composé préparé à l'exemple 3, dissous dans le t-butanol selon un rapport molaire: composé exemple 2/t-BuOH/K/composé exemple 3 1/40/5/5, on obtient le dérivé 6b qui présente les caractéristiques suivantes:

Rendement: 20%; TF: 25 °C; $^1H$ RMN ($CCl_4$): 1,15 (m, 92H, –$CH_2$–), 3,30 (m, 18H, –$OCH_2$– et –CHO), 4,45 (s, 4H, $\varnothing CH_2$–), 7,45 (s, 2H, arom.).

Exemples 6 à 8 – Dérivés 6c à 6e

Ces exemples concernent la préparation de différents dérivés obtenus comme cela est décrit à l'exemple 4 mais en remplaçant le 1-dodécanol par différents étheralcools.

Les alcools monométhyléther polyéthylèneoxyde qui sont commercialement disponibles et vendus par la société Polysciences présentent une polydispersion très importante.

L'alcool ayant un poids moléculaire d'environ 350 a été analysé par chromatographie en phase gazeuse après silylation des groupes hydroxy terminaux.

On obtient ainsi la composition suivante:

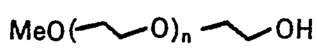

n=1, 0,3%; n=2, 12,2%; n=3; 12,7%;
n=4, 15,9%; n=5, 10,6%; n=6, 22,2%;
n=7, 19,6%; n=8, 5,3%; n=9, 1,3%.

Les alcools suivants ont pu être purifiés:

– l'alcool dans lequel n=1 (35–50 °C; 66,5 Pa (0,5 Torr))

– l'alcool dans lequel n=7 (200 °C; 1,33 Pa ($10^{-2}$ Torr)).

L'alcool dans lequel n=14 a été purifié par chromatographie sur $SiO_2$ (éluent: $CHCl_3$/MeOH, 95/5) après recristallisation dans l'éther.

Ces alcools sont traités comme dans l'exemple 4 et permettent d'obtenir respectivement les composés 6c à 6e dont les caractéristiques sont les suivantes:

6c: n=1

Rendement: 40%; $^1H$ RMN ($CCl_4$): 3,15 (s, 6H, –$OCH_3$), 3,35 (s, 8H, –$CH_2O$–), 3,45 (s, 8H, –$CH_2O$–), 4,30 (s, 4H, arom. $CH_2O$–), 7,40 (s, 2H, arom). $^{13}C$ RMN ($CDCl_3$): 58,9, 69,4, 69,9, 70,47, 70,51, 71,9, 123,4, 133,1, 137,4.

Analyse: $C_{18}H_{28}O_6Br_2$ (500,24):
Calculé (%): C 43,22; H 5,64; O 19,19; Br 31,95;
Trouvé (%): C 43,31; H 5,56; O 19,25; Br 32,39;
total: 100,51%.

6d: n=7

Rendement: 40%; $^1H$ RMN ($CCl_4$): 3,15 (s, 6H, –$OCH_3$), 3,40 (s, 64H, –$CH_2O$–), 4,3 (s, 4H, arom. –$CH_2O$–), 7,45 (s, 2H, arom). $^{13}C$ RMN ($CDCl_3$): 58,9, 69,5, 70,6, 71,9, 123,5, 133,2, 137,4.

Analyse: $C_{42}H_{76}O_{18}Br_2$ (1028,88):
Calculé (%): C 49,03; H 7,44; O 27,99; Br 15,53;
Trouvé (%): C 49,07; H 7,51; O 27,99; Br 15,76;
total: 100,33%.

6e: n=14

Rendement: 10%; $^1H$ RMN ($CCl_4$): 3,15 (s, 6H, –$OCH_3$), 3,45 (s, 120H, –$CH_2O$–), 4,35 (s, 4H, arom. –$CH_2O$–), 7,45 (s, 2H, arom.).

Analyse: $C_{70}H_{132}O_{32}Br_2$ (1645,62):
Calculé (%): C 51,9; H 8,08; O 31,11; Br 9,71;
Trouvé (%): C 51,28; H 8,18; O 31,51; Br 9,21;
total: 100,18%.

Exemples 9 à 12 – Dérivés 1,2-dicyano-4,5-bis(alcoxyméthyl)benzène (7a–7e)

Les différents composés sont préparés à partir des dérivés dibromés des exemples 4 à 7.

1 mole du dérivé dibromé préparé aux exemples précédents est dissoute dans le DMF (52 moles) et traitée avec CuCN (3 moles). Le mélange est chauffé à reflux et sous atmosphère d'azote pendant 6 heures. Les progrès de la réaction sont contrôlés par CCM ($SiO_2$, éluent: $CHCl_3/Et_2O$, 97,5/2,5). S'il reste du composé de départ, on ajoute une nouvelle quantité de CuCN et le reflux est maintenu pendant quelques heures supplémentaires. Le mélange réactionnel est filtré à chaud et le filtrat est évaporé jusqu'à siccité. Le résidu est traité avec une solution d'éthylène-diamine dans l'eau (1/3, v/v). La phase aqueuse est alors extraite avec $CHCl_3$. Les extraits organiques combinés sont rincés avec de l'eau distillée, séchés sur $Na_2SO_4$ et évaporés jusqu'à siccité.

Le résidu huileux verdâtre est alors purifié par chromatographie sur $SiO_2$ avec les éluents suivants en fonction du produit:

7a: $CHCl_3$; 7b: $CHCl_3$/toluène (75/25); 7c: $CHCl_3$/MeOH (99,5/0,5); 7d: $CHCl_3$/MeOH (96/4).

7a:

Rendement: 40%; TF: 82 °C; $^1H$ RMN ($CCl_4$): 1,2 (s, 46H, –$CH_2$–), 4,15 (t, 4H, –$CH_2O$–), 4,35 (s, 4H, arom. –$CH_2O$–), 7,6 (s, 2H, arom.). $^{13}C$ RMN ($CDCl_3$): 14,0 22,6, 26,1, 29,26, 29,35, 29,5, 31,8, 68,4, 71,6, 114,3, 115,5, 131,8, 142,5.

Analyse: $C_{34}H_{56}O_2N_2$ (524,84):
Calculé (%): C 77,81; H 10,76; O 6,10; N 5,34;
Trouvé (%): C 77,82; H 10,91; O 6,06; N 5,16;
total: 99,95%.

7b:

Rendement: 40%; $^1H$ RMN ($CDCl_3$): 1,15 (s, 92H, –$CH_2$–), 3,25 (m, 18H, –$CH_2O$–, –CHO–), 4,55 (s, 4H, arom. –$CH_2O$–), 7,7 (s, 2H, arom.). $^{13}C$ RMN ($CDCl_3$): 14,1, 22,7, 26,2, 29,4, 29,5, 29,7, 31,9, 68,0, 71,2, 71,8, 78,7, 114,2, 115,8, 132,0, 142,9.

7c:

Rendement: 60%; $^1H$ RMN ($CCl_4$): 3,1 (s, 6H, –$OCH_3$), 3,3 (s, 8H, –$OCH_2$–), 3,5 (s, 8H,

$-OCH_2-$), 4,45 (s, 4H, arom. $-CH_2O-$), 7,70 (s, 2H, arom.). $^{13}C$ RMN ($CDCl_3$): 59,0, 68,9, 70,6, 71,9, 114,4, 115,6, 132,0, 142,6.
Analyse: $C_{20}H_{26}O_6N_2$ (392,45):
Calculé (%): C 61,21; H 7,19; O 24,46; N 7,13;
Trouvé (%): C 59,93; H 7,08; O 25,59; N 7,10;
total: 99,7%.

7d:
Rendement: 60%; $^1H$ RMN ($CCl_3$): 3,1 (s, 6H, $-OCH_3-$), 3,35 (s, 64H, $-OCH_2-$), 4,45 (s, 4H, arom. $-CH_2O-$), 7,70 (s, 2H, arom.).
Analyse: $C_{44}H_{76}O_{18}N_2$ (921,1):
Calculé (%): C 57,77; H 8,32; O 31,27; N 3,04;
Trouvé (%): C 56,66; H 8,40; O 32,24; N 2,70;
total: 97,3%.

Exemple 13 – Phtalocyanines octasubstituées (1a–1e)

Les phtalocyanines des composés obtenus aux exemples précédents peuvent être obtenues directement en traitant le dérivé dicyané dans le N,N-diméthylamino-2-propanol en présence d'ammoniaque ou éventuellement en convertissant le dérivé dicyané dans le dérivé correspondant diimino-isoindole qui est ensuite retraité à reflux dans le N,N-diméthylamino-éthanol.

Le second procédé permet d'obtenir des résultats plus reproductibles.

Préparation du 2, 3, 9, 10, 16, 17, 23, 24-octa-(dodécyloxy-1-méthyl)- 29H,31H-phtalocyanine (1a)

1,5 g (2,86 mmoles) de 7a est dissous dans 1,5 ml de N,N-diméthylamino-2-propanol fraîchement distillé (point d'ébullition: 120–127 °C). De l'ammoniac est introduit sous forme gazeuse à travers le milieu réactionnel qui est alors chauffé sous reflux pendant 72 heures. La solution devient rapidement bleu-vert. Le solvant est évaporé et le résidu verdâtre est extrait avec de l'éthanol chaud pour éliminer les traces restantes de solvant et la matière de départ n'ayant pas réagi. La recristallisation à partir d'heptane fournit des microcristaux vert foncé.

Le rendement est de 40 à 60% et varie sans que l'on ait pu mettre en évidence l'influence de paramètres tels que la pureté du solvant, la température, par exemple.

Le composé 1a obtenu présente les caractéristiques suivantes:

1a:
TF: 78 °C (mésophase discotique); $^1H$ RMN ($CCl_4$):
1,1 (m, 184H, $-CH_3$ et $-CH_2-$), 3,3 (bt, 12H, $-CH_2O-$), 4,75 (bs, 12H, arom. $-CH_2O-$), 8,35 (bs, 8H, arom.).
Analyse: $C_{136}H_{226}O_8N_8$ (2101,36):
Clculé (%): C 77,74; H 10,84; O 6,09; N 5,35;
Trouvé (%): C 77,68; H 10,78; O 6,37; N 5,62;
total: 100,45%.

Exemple 14 – Complexe de cuivre du dérivé 1a

50 mg (24 μmoles) du composé 1a obtenu à l'exemple 13 sont dissous sous azote dans 5 ml de n-pentanol distillé (46 mmoles). Le mélange réactionnel est agité et chauffé à 100 °C. On ajoute alors 0,52 ml d'une solution de potassium dans le 1-pentanol (0,138 mole); après 1 heure, on ajoute 9,6 mg (71 μmoles) de $CuCl_2$ anhydre en poudre et la réaction est poursuivie pendant 2 heures. Le solvant est alors évaporé jusqu'à siccité et le résidu bleuâtre est filtré à travers une colonne d'alumine (éluent: toluène) pour éliminer l'excès de sel métallique. Le complexe de cuivre est recristallisé à partir d'heptane et présente les caractéristiques suivantes:
Rendement: 100%; TF: 70 °C (mésophase discotique).
Analyse: $C_{136}H_{224}O_8N_8Cu$ (2162,88):
Calculé (%): C 75,52; H 10,44; O 5,92; N 5,18; Cu 2,94;
Trouvé (%): C 75,19; H 10,08; O 5,91; N 5,77; Cu 3,05;
total: 100%.

En suivant ce procédé il est possible de préparer d'autres complexes métalliques en particulier le complexe métallique avec le zinc qui présente les caractéristiques suivantes:
1a, Zn:
Analyse: $C_{136}H_{224}O_8N_8Zn$ (2164,71):
Calculé (%): C 75,46; H 10,43;
Trouvé (%): C 75,14; H 10,29;

D'autres dérivés métalliques ont été préparés, en particulier des dérivés d'étain, de manganèse, de platine et de palladium.

Exemple 15 – 2,3,10,16,17,23,24-octa-(1-méthoxy-3-oxapentaoxy- 5-méthyl)-29H,31H-phtalocyanine (1c)

Le dérivé diimino-isoindole est formé en traitant 500 mg (1,27 mmole) du dérivé dicyano 6c dans 2,5 ml de méthanol anhydre avec 0,69 ml d'une solution de méthanolate de sodium (0,037 M). On fait alors buller de l'ammoniac dans le mélange réactionnel à la température ambiante pendant 1 heure, puis on chauffe à reflux pendant 4 heures supplémentaires. Lorsque la réaction est terminée le solvant est avaporé jusqu'à siccité et le résidu est filtré à travers une colonne de $SiO_2$ (éluent: MeOH). Le résidu huileux verdâtre obtenu est utilisé sans être purifié.

La phtalocyanine correspondante est formée en chauffant à reflux et sous azote 520 mg (1,27 mmole) du diimino-isoindole obtenu précédemment dans 1 ml de N,N-diméthylamino-éthanol fraîchement distillé. Le reflux est maintenu pendant 7 heures. Le solvant est alors évaporé à siccité et le résidu est purifié par chromatographie sur $SiO_2$ (éluent: $CHCl_3$/MeOH, 98/2).

Le produit obtenu présente les caractéristiques suivantes:
Rendement: 40%, huile visqueuse; $^1H$ RMN ($CCl_4$): 3,2 (s, 24H, $-OCH_3$), 3,5 (bs, 32H, $-CH_2O-$), 3,6 (bs, 32H, $-CH_2O-$), 4,4 (bs, 16H, arom. $CH_2O-$), 7,65 (bs, 8H, arom.).
Analyse: $C_{80}H_{114}O_{24}N_8$ (1571,83):
Calculé (%): C 61,13; H 7,31; O 24,43; N 7,13;

Trouvé (%): C 59,87; H 7,06; O 23,93; N 7,16; total: 98,02%.

Exemple 16 – Préparation du dérivé de Pb du composé 1a

Le produit de départ est le composé 1a de l'exemple 13.

88 µmoles de ce composé sont dissoutes dans 15 ml d'alcool amylique anhydre. La solution sous azote est portée à 100 °C avec agitation magnétique. Après dissolution totale, on ajoute 1,5 ml d'une solution d'amylate de potassium 0,55 molaire (850 µmoles) dans l'alcool amylique. On ajoute alors un large excès de nitrate de plomb anhydre (0,5 g). On laisse réagir 15 à 30 minutes à 100 °C. On contrôle le degré d'avancement de la réaction par chromatographie sur couche mince (éluant: $Et_2O/CHCl_3$ 10/90; $(C_{12})_8Pc$ Pb rf: 0,45).

On laisse revenir à température ambiante, on filtre sur coton de verre et le filtrat est versé dans du méthanol; le dérivé de Pb précipite. Le précipité est séparé, filtré rapidement sur colonne d'alumine et recristallisé dans l'acétate d'éthyle. Sa structure est représentée à la figure 2. (Rendement (74%).

Analyse: $C_{136}H_{224}O_8N_8Pb$:
Calculé (%): C 70,80; H 9,81; O 5,55; N 4,86; Pb 8,98;
Trouvé (%): C 70,75; H 10,44; O N 4,84; Pb 8,92.

La structure de ce composé est représentée figure 2.

Exemple 17 – Préparation de $(C_{12}OCH_2)_6(CN)_2Pc\,H_2$

Le dérivé tétracyané (250 mg, 1,4 mmole) est dissous dans 10 ml de pentanol à 80 °C. De l'ammoniac est bullé à travers la solution; après 10 minutes, on ajoute 0,25 ml d'amylate de sodium (0,19 mole) dans l'alcool amylique. On revient à température ambiante et on laisse agiter 2 heures.

On porte à 60 °C 2 autres heures puis 1 heure à 100 °C. On filtre à chaud et on rince deux fois à l'éthanol, on sèche sous vide au dessicateur.

Poids moléculaire: 195,18
Analyse:
Calculé: C 61,54; H 2,58; O 3,55; N 35,88;
Trouvé : C 58,93; H 2,90; N 34,62

50 mg (9,2 µmoles) du composé de l'exemple 14 plus 6 mg (3,06 µmoles) du composé précédent sont mis en suspension dans 70 µl de N,N-diméthyléthanolamine, le mélange est purgé à l'azote et on porte sous reflux 7 heures. On évapore ensuite à sec. Onpurifie par chromatographie sur $SiO_2$ (éluant: 0,1% $Et_2O/CHCl_3$) puis par une seconde chromatographie sur $SiO_2$ (5% $Et_2O$/30% $CHCl_3$/65% hexane).

Rendement 20%.
Analyse: $C_{112}H_{172}O_6N_{10}$:
Calculé: C 76,67; H 9,88; O 5,47; N 7,98;
Trouvé : C 76,16; H 10,10; O 5,71; N 7,84.

Exemple 18 – Préparation du dérivé de Lu du composé de l'exemple 14

50 mg du composé de l'exemple 14 sont dissous à 140 °C dans 1 ml de 1,2-propanediol. On additionne alors 0,5 ml d'une solution 0,143 molaire d'amylate de potassium dans l'alcool amylique. 25 mg d'acétate de lutecium dissous dans 1 ml de 1,2-propanediol sont alors ajoutés. La réaction terminée, on évapore à sec et on chromatographie sur silice (éluant: 5% AcOEt/heptane).

Rendement: 40%
Analyse: $C_{272}H_{448}O_{16}N_{16}Lu$ (4373,65):
Calculé: C 74,70; H 10,32; N 5,12;
Trouvé : C 74,38; H 10,80; N 4,51.

La structure de ce composé est représentée à la figure 3. Il sera nommé ci-après $[(C_{12})_8Pc]_2Lu$.

Exemple 19

En remplaçant dans l'exemple 4 le 1-dodécanol par l'alcool caprylique on obtient finalement, après les traitements correspondant aux exemples 5, 9 à 12, 13 et 18, le dérivé If, c'est-à-dire le dérivé de lutécium du composé 1f qui sera nommé ci-après $[(C_8)_8Pc]_2Lu$.

Exemple 20 – Caractéristiques de $[(C_{12})_8Pc]_2Lu$

$[(C_{12})_8Pc]_2Lu$ présente les caractéristiques suivantes:

Les cristaux solides obtenus après chromatographie fondent à 30 °C vers un état isotrope:

$$K \xrightarrow{30\,^\circ C} I$$

K: cristal
I: isotrope

Par refroidissement, une transition est observable par ATD à 6 °C; un nouveau chauffage conduit à une transition à 24 °C suivie d'un état isotrope à 30 °C soit:

$$I \xrightarrow{6\,^\circ C} K,$$

$$K' \xrightarrow{24\,^\circ C} CL \xrightarrow{30\,^\circ C} I$$

CL: cristal liquide

Une phase métastable cristalline liquide est observable entre 24 et 30 °C.

Les figures 4 à 6 représentent les courbes d'analyse thermique différentielle obtenues (ATD) avec $[(C_{12})_8Pc]_2Lu$:

– figure 4: premier chauffage à partir de l'état cristallin,

– figure 5: refroidissement à partir de l'état isotrope,

– figure 6: transition à 24 °C obtenue sur le produit chauffé à 30 °C puis refroidi à 6 °C; cette dernière transition est la seule qui subsiste dans toutes les expériences ultérieures d'ATD.

$[(C_{12})_8Pc]_2Lu$ peut être oxydé soit électrochimiquement, soit chimiquement ($H_2O_2$:$H_2SO_4$). Un composé rougeâtre est alors obtenu. Les transitions observables par microscopie en lumière

polarisée sont alors fortement altérées par rapport à l'état initial:

$$\text{CL (produit rouge)} \xrightarrow{\sim 110\ °C} \text{I}$$

Cette dernière température est dépendante du taux d'oxydation du produit. Plus le taux d'oxydation est faible plus la température de transition vers l'état isotrope se rapproche de 30 °C.

$[(C_8)_8Pc]_2Lu$ a également été étudié. Les propriétés cristallines liquides sont plus faciles à étudier que pour le composé à chaînes dodécyles:

chauffage puis température ambiante: CL

$$\text{CL} \xrightarrow{40\ °C} \text{I} \xrightarrow{31\ °C} \text{CL}$$

Etude en résonance paramagnétique électronique (RPE)

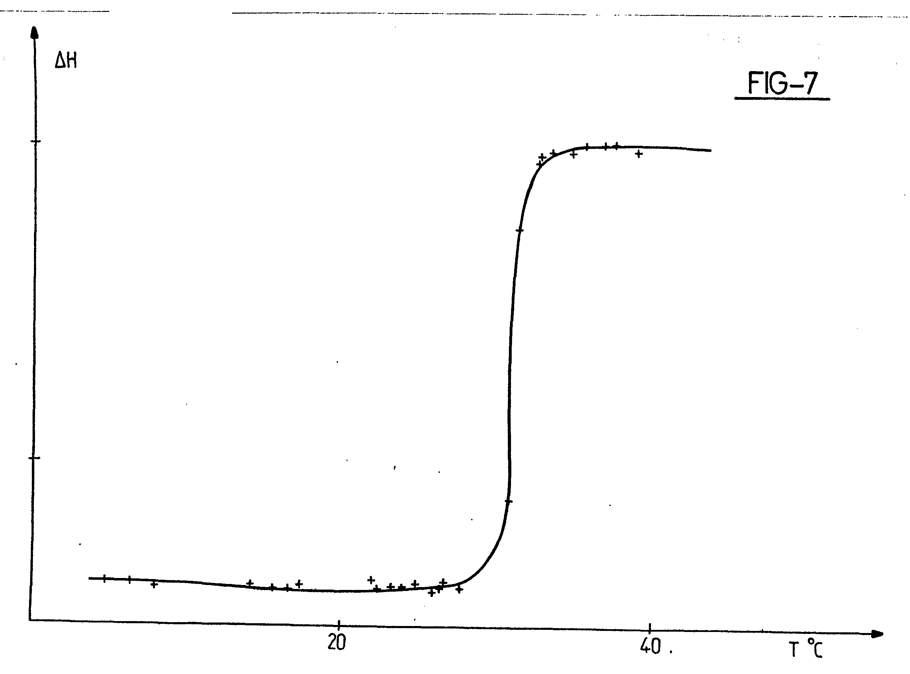

Dans tous les cas étudiés, l'intégration du signal RPE permet de calculer que la forme verte, celle initialement formée, comporte 1 spin par molécule de diphtalocyanine de lutécium avec g $\sim$ 2,0 proche de l'électron libre. Il est possible de suivre les différentes transitions K→Cl→I en observant la largeur de raie RPE en fonction de la température au voisinage des points de transition comme cela est représenté figure 7.

Il n'a pas été noté de signal correspondant à $M = \pm 2$ ni dans la phase cristalline (5,3 °C) ni dans la phase isotrope (31,9 °C). La loi de Curie-Weiss est obéie au-dessus de 100°K; par contre, une transition vers un état antiferromagnétique est observée (température de transition 87°K). Il faut noter que pour le composé non substitué, la loi de Curie est observée entre 5 et 100°K.

Des essais effectués on a pu mettre en évidence que les composés étudiés:

- formaient des phases cristallines liquides (texture en microscopie en lumière polarisée et mesures d'analyse thermique différentielle),
- changeaient de couleur – du vert vers le rougeâtre – électrochimiquement ou chimiquement.

Les différentes caractérisations physicochimiques (microanalyses, RMN $^{13}C$ et $^{1}H$, RPE) sont en accord avec les structures proposées.

**Revendications**

1. Composé organométallique de formule:

$$[(R')_n (R'')_m Por]_p Me \qquad (I)$$

dans laquelle:

- Por représente un radical choisi parmi les radicaux:
- phtalocyanine,
- porphyrine,
- tétraazaporphyrine,
- tétra(naphto)tétraazaporphyrine,
- tétra(phénanthro)tétraazaporphyrine,
- R' et R'' représentent des radicaux identiques ou différents, fixés sur les atomes de carbone périphériques du cycle Por;
- R' est un radical à caractère aliphatique dont la chaîne carbonée peut être interrompue par un ou plusieurs hétéroatomes choisis parmi O, S, N et Se ou par un radical phénylène, la chaîne carbonée contenant au moins 6 atomes;
- R'' est un radical cyano ou halogéno;
- n et m sont des nombres entiers dont la somme est au moins égale à 4 et au plus égale au nombre d'atomes de carbone périphériques du cycle Por;
- n au moins égal à 4,
- p est égal à 1 ou 2;
- Me est un métal.

avec la précision que quand p est égal à 1, Me est choisi dans le groupe constitué par le groupe des lanthanides et de l'yttrium, l'uranium, le thorium, l'étain, le platine, le palladium, le manganèse et le plomb; et lorsque Por est une tétra(naphto)-tétraazaporphyrine, n est supérieure à 4.

2. Composé dissymétrique organométallique de formule générale:

$$[(R')_n (R'')_m Por]_p Me \qquad (I)$$

dans laquelle:

- Por représente un radical choisi parmi les radicaux:
- phtalocyanine,
- porphyrine,
- tétraazaporphyrine,
- tétra(naphto)tétraazaporphyrine,
- tétra(phénanthro)tétraazaporphyrine,
- R' et R'' représentent des radicaux identiques ou différents, fixés sur les atomes de carbone périphériques du cycle Por;
- R' est un radical à caractère aliphatique dont la chaîne carbonée peut être interrompue par un ou plusieurs hétéroatomes choisis parmi O, S, N et Se ou par un radical phénylène, la chaîne carbonée contenant au moins 6 atomes;
- R'' est un radical cyano ou halogéno;
- n et m sont des nombres entiers dont la somme est au moins égale à 4 et au plus égale au nombre d'atomes de carbone périphériques du cycle Por;
- n au moins égal à 4,
- p est égal à 1 ou 2;
- Me est un métal.

3. Composé selon les revendications 1 et 2 prises séparément, de formule:

$$[(R)_n Pc]_p Me$$

dans laquelle:

Pc symbolise le radical phtalocyanine substitué suivant:

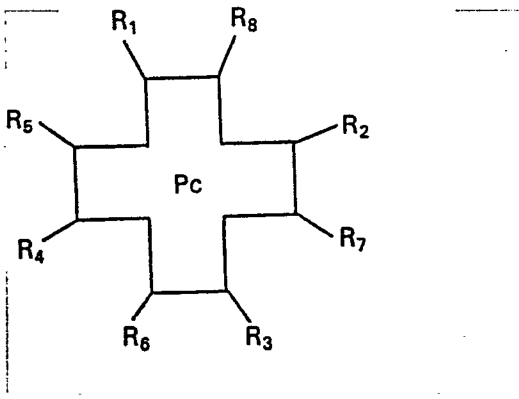

(I')

$R_1$ à $R_8$ représentent H ou l'une des valeurs données pour R' et R", sous réserve qu'au moins 4 des radicaux $R_1$ à $R_8$ soient des radicaux aliphatiques.

4. Composé selon la revendication 3, caractérisé en ce que $R_1$ à $R_8$ représentent H ou un radical choisi parmi les radicaux alcoyles en $C_6$ à $C_{50}$, les radicaux alcoyles en $C_6$ à $C_{50}$, comportant un ou plusieurs hétéroatomes choisis parmi O, S ou N dans la chaîne.

5. Composé selon la revendication 3, caractérisé en ce que $R_1$ et $R_8$ représentent le radical cyano.

6. Composé selon l'une des revendications 3 et 4, caractérisé en ce que les radicaux $R_1$ à $R_8$ sont des radicaux aliphatiques identiques.

7. Composé selon l'une des revendications 3 et 4, caractérisé en ce que les radicaux $R_1$ à $R_8$ représentent H ou $-CH_2-O-R_{10}$, $R_{10}$ étant un radical alcoyle en $C_4$ à $C_{50}$ ou une chaîne alcoylée interrompue par un ou plusieurs hétéroatomes choisis parmi O, S ou N.

8. Composé selon la revendication 7, caractérisé en ce que $R_{10}$ est choisi parmi:
$-C_8H_{17}$; $-C_{12}H_{25}$; $-CH(-CH_2-O-C_{12}H_{25})_2$;

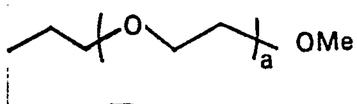 a = 1,7 ou 14

9. Composé selon la revendication 8, caractérisé en ce que le radical phtalocyanine est représenté par une des formules suivantes

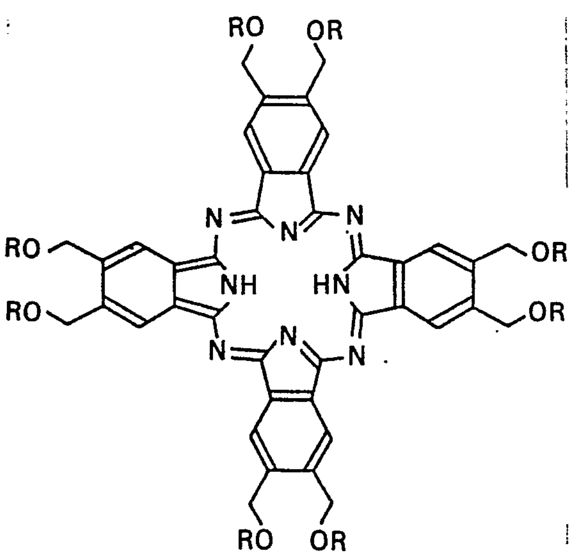

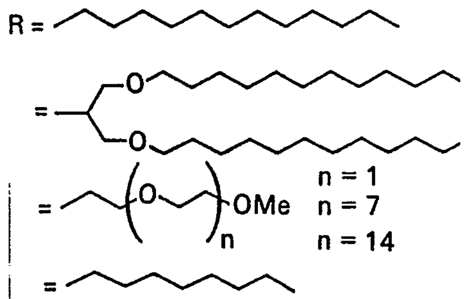

n = 1 n = 7 n = 14

$1_a$ (Ia)
$1_b$ (Ib)
$1_c$ (Ic)
$1_d$ (Id)
$1_e$ (Ie)
$1_f$ (If)

10. Composé selon l'une des revendications 1 à 9, caractérisé en ce que le métal est choisi parmi le groupe des lanthanides et de l'yttrium, l'uranium, le thorium, l'étain et le plomb.

11. Composé selon la revendication 10, caractérisé en ce que le métal choisi est le lutécium.

12. Application des composés selon l'une des revendications 1 à 11, à l'électronique ou à l'optoélectronique.

13. Application selon la revendication 12 à la réalisation d'écrans de visualisation à commande électronique.

14. Procédé de préparation de composés selon l'une des revendications 1 à 11, caractérisé en ce que l'on fait réagir un composé de formule: $(R')_n(R'')_m$Por sous forme de dianion avec un sel métallique du métal Me.

15. Procédé selon la revendication 14, caractérisé en ce que le dianion du composé $(R')_n(R'')_m$Por est obtenu par action d'un alcoolate de métal alcalin.

## Patentansprüche

1. Organometallische Verbindung der Formel

$$[(R')_n (R'')_m Por]_p Me \quad (I)$$

in der bedeuten:

- Por einen Rest, ausgewählt aus:
- Phthalocyanin,
- Porphyrin,
- Tetraazaporphyrin,
- Tetra(naphtho)tetraazaporphyrin und
- Tetra(phenanthren)tetraazaporphyrin,
- R' und R" gleiche oder verschiedene Reste, die an periphere Kohlenstoffatome des durch Por wiedergegebenen Ringes gebunden sind;
- R' einen Rest aliphatischen Charakters, dessen Kohlenstoffkette durch ein oder mehrere Heteroatome, ausgewählt aus O, S, N und Se oder durch einen Phenylenrest unterbrochen sein kann, wobei die Kohlenstoffkette mindestens 6 Atome aufweist;
- R" einen Cyano- oder Halogenrest;
- n und m ganze Zahlen, deren Summe mindestens gleich 4 ist und höchstens gleich ist der Anzahl von peripheren Kohlenstoffatomen des durch Por dargestellten Ringes;
- n wenigstens gleich 4;
- p gleich 1 oder 2;
- Me ein Metall,

wobei gilt, dass, wenn p gleich 1 ist, Me ausgewählt ist aus der Gruppe bestehend aus der

Gruppe der Lanthaniden und des Yttriums, dem Uran, dem Thorium, dem Zinn, dem Platin, dem Palladium, dem Mangan und dem Blei; und dass, wenn Por ein Tetra(naphtho)-tetraazaporphyrinring ist, n grösser als 4 ist.

2. Unsymmetrische organometallische Verbindung der allgemeinen Formel:

$$[(R')_n (R'')_m Por]_p Me \qquad (I)$$

in der bedeuten:
- Por einen Rest, ausgewählt aus:
- Phthalocyanin,
- Porphyrin,
- Tetraazaporphyrin,
- Tetra(naphtho)tetraazaporphyrin und
- Tetra(phenanthren)tetraazaporphyrin,
- R' und R'' gleiche oder verschiedene Reste, die an periphere Kohlenstoffatome des durch Por wiedergegebenen Ringes gebunden sind;
- R' einen Rest aliphatischen Charakters, dessen Kohlenstoffkette durch ein oder mehrere Heteroatome, ausgewählt aus O, S, N und Se oder durch einen Phenylenrest unterbrochen sein kann, wobei die Kohlenstoffkette mindestens 6 Atome aufweist;
- R'' einen Cyano- oder Halogenrest;
- n und m ganze Zahlen, deren Summe mindestens gleich 4 ist und höchstens gleich ist der Anzahl von peripheren Kohlenstoffatomen des durch Por dargestellten Ringes;
- n wenigstens gleich 4,
- p gleich 1 oder 2;
- Me ein Metall.

3. Verbindung nach Ansprüchen 1 und 2, jeweils entsprechend der Formel:

$$[(R)_n Pc]_p Me$$

in der bedeuten:

Pc einen wie folgt substituierten Phthalocyaninrest:

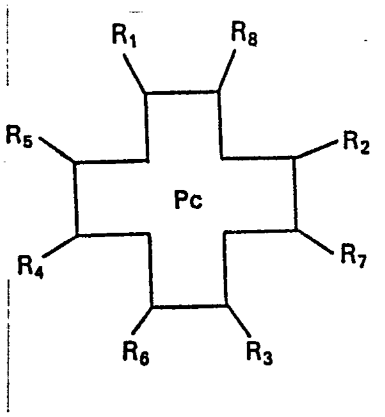

(I')

wobei $R_1$ bis $R_8$ für H oder einen der für R' und R'' angegebenen Reste stehen, wobei gilt, dass mindestens 4 der Reste $R_1$ bis $R_8$ aliphatische Reste sind.

4. Verbindung nach Anspruch 3, dadurch gekennzeichnet, dass $R_1$ bis $R_8$ für H oder Reste, ausgewählt aus Alkylresten mit 6 bis 50 C-Atomen und Alkylresten mit 6 bis 50 C-Atomen, die ein oder mehrere Heteroatome, ausgewählt aus O, S oder N in der Kette aufweisen, stehen.

5. Verbindung nach Anspruch 3, dadurch gekennzeichnet, dass $R_1$ und $R_8$ für Cyanoreste stehen.

6. Verbindung nach einem der Ansprüche 3 und 4, dadurch gekennzeichnet, dass die Reste $R_1$ bis $R_8$ für identische aliphatische Reste stehen.

7. Verbindung nach einem der Ansprüche 3 und 4, dadurch gekennzeichnet, dass die Reste $R_1$ bis $R_8$ für H oder einen Rest der Formel $-CH_2-O-R_{10}$ stehen, worin $R_{10}$ ein Alkylrest mit 4 bis 50 C-Atomen ist oder eine Alkylkette, die durch ein oder mehrere Heteroatome, ausgewählt aus O, S oder N, unterbrochen ist.

8. Verbindung nach Anspruch 7, dadurch gekennzeichnet, dass $R_{10}$ aus folgenden Resten ausgewählt ist:

$-C_8H_{17}; -C_{12}H_{25}; -CH(-CH_2-O-C_{12}H_{25})_2;$

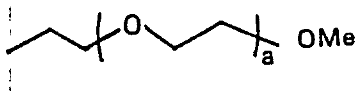 a = 1,7 ou 14

9. Verbindung nach Anspruch 8, dadurch gekennzeichnet, dass der Phthalocyaninrest einer der folgenden Formeln entspricht:

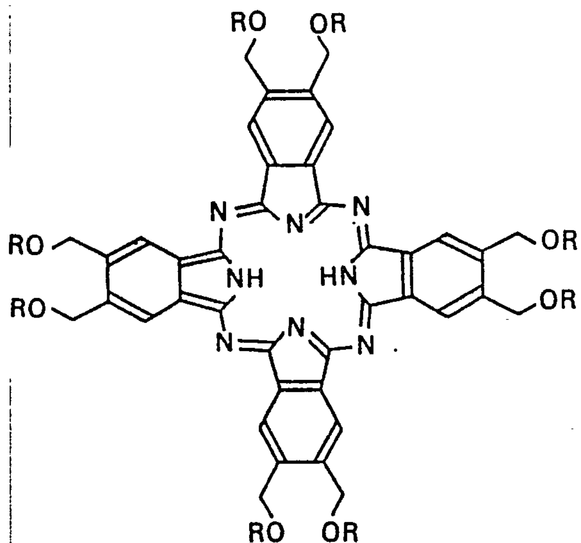

R = 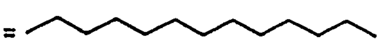 $1_a$ (Ia)

= 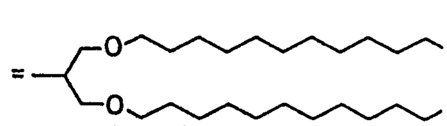 $1_b$ (Ib)

= 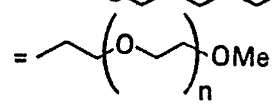 n = 1 $1_c$ (Ic); n = 7 $1_d$ (Id); n = 14 $1_e$ (Ie)

= 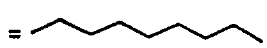 $1_f$ (If)

10. Verbindung nach einem der Ansprüche 1 bis 9, dadurch gekennzeichnet, dass das Metall ausgewählt ist unter der Gruppe der Lanthaniden und des Yttriums, dem Uran, dem Thorium, dem Zinn und dem Blei.

11. Verbindung nach Anspruch 10, dadurch gekennzeichnet, dass das Metall Lutetium ist.

12. Anwendung der Verbindungen nach einem der Ansprüche 1 bis 11 auf dem Gebiet der Elektronik und Optoelektronik.

13. Anwendung nach Anspruch 12 zur Herstellung eines Bildschirmes mit elektronischer Steuerung.

14. Verfahren zur Herstellung von Verbindungen nach einem der Ansprüche 1 bis 11, dadurch gekennzeichnet, dass man eine Verbindung der Formel: $(R')_n(R'')_m$Por unter Bildung des Dianions mit einem Metallsalz des Metalles Me umsetzt.

15. Verfahren nach Anspruch 14, dadurch gekennzeichnet, dass man das Dianion der Verbindung $(R')_n(R'')_m$Por durch Einwirkung eines Alkalimetallalkoholates gewinnt.

## Claims

1. Organo-metallic composition of the formula:

$$[(R')_n(R'')_m \, Por]_p Me \qquad (I)$$

in which:

- Por represents a radical selected from amongst the following radicals:
  - phthalocyanine,
  - porphyrine,
  - tetraazaporphyrine,
  - tetra(naphtho)tetraazaporphyrine,
  - tetra(phenanthro)tetraazaporphyrine,
- R' and R'' represent identical or different radicals attached to the peripheral carbon atoms of the Por ring;
- R' is a radical of aliphatic character of which the carbon chain may be interrupted by one or several hetero-atoms selected from amongst O, S, N and Se or by a phenylene radical, the carbon chain containing at least six atoms;
- R'' is a cyano or halo radical;
- n and m are integers, the sum of which is equal to at least 4 and at most equal to the number of peripheral carbon atoms in the Por ring;
- n is equal to at least 4,
- p is equal to 1 or 2
- Me is a metal.

with the limitation that when p is equal to 1, Me is selected from the group constituted by the group of lanthanides and yttrium, uranium, thorium, tin, platinium, palladium, manganese and lead; and when Por is a tetra (naphtho) tetraazaporphyrine n is greater than 4.

2. Asymmetrical organo-metallic composition of the general formula:

$$[(R')_n(R'')_m \, Por]_p \, Me \qquad (I)$$

in which:

- Por represent a radical selected from amongst the following radicals:
  phthalocyanine
  porphyrine
  tetraazaporphyrine
  tetra(naphtho)tetraazaporphyrine
  tetra(phenanthro)tetraazaporphyrine
- R' and R'' represents identical or different radicals, attached to the peripheral carbon atoms of the Por ring;
- R' is a radical of aliphatic character of which the carbon chain may be interrupted by one or several hetero-atoms selected from amongst O, S, N and Se or by a phenylene radical, the carbon chain containing at least 6 atoms;
- R'' is a cyano or halo radical;
- n and m are integers, the sum of which is equal to at least 4 and at most equal to the number of peripheral carbon atoms in the Por ring;
- n is equal to at least 4
- p is equal to 1 or 2;
- Me is a metal.

3. Composition according to claims 1 and 2 taken separately, of the formula:

$$[(R)_n Pc]_p \, Me$$

in which:

Pc symbolises the following substituted phthalocyanine radical:

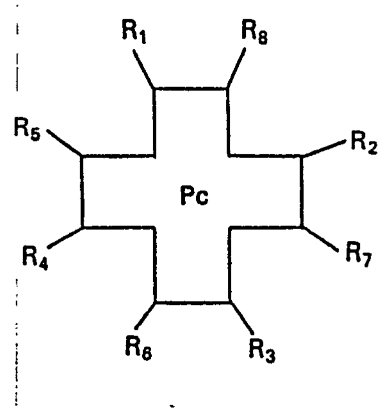

(I')

$R_1$ to $R_8$ representing H or one of the values given for R' and R'', with the proviso that at least 4 of the radicals $R_1$ to $R_8$ are aliphatic radicals.

4. Composition according to claim 3, characterised in that $R_1$ to $R_8$ represent H or a radical selected from among the alkyl radicals from $C_6$ to $C_{50}$, the alkyl radicals from $C_6$ to $C_{50}$ containing one or several hetero-atoms selected from amongst O, S or N in the chain.

5. Composition according to claim 3, characterised in that $R_1$ and $R_8$ represent the cyano radical.

6. Composition according to one of claims 3 and 4, characterised in that the radicals $R_1$ to $R_8$ are identical aliphatic radicals.

7. Composition according to one of claims 3 and 4, characterised in that the radicals $R_1$ to $R_8$ represent H or $-CH_2-O-R_{10}$, $R_{10}$ being an alkyl radical from $C_4$ to $C_{50}$ or an alkylated chain interrupted by one or several hetero-atoms selected from amongst O, S or N.

8. Composition according to claim 7, characterised in that $R_{10}$ is selected from amongst:

$-C_8H_{17}$; $-C_{12}H_{25}$; $-CH(-CH_2-O-C_{12}H_{25})_2$;

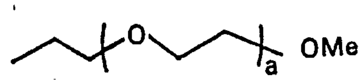

a = 1,7 ou 14

9. Composition according to claim 8, characterised in that the phthalocyanine radical is represented by one of the following formulae:

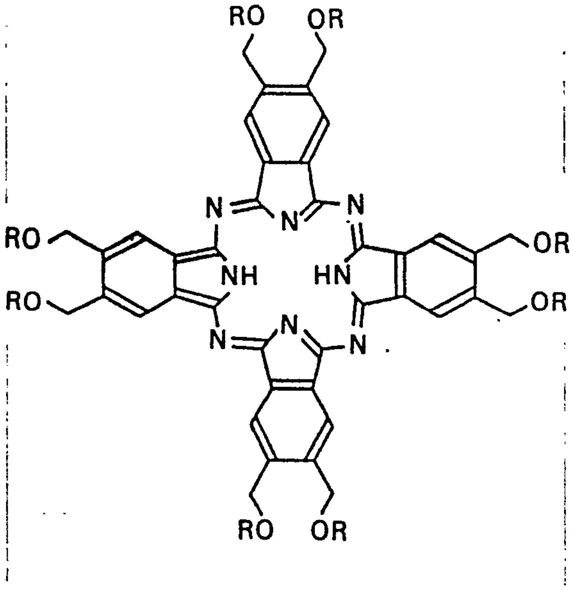

R = 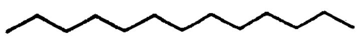 $1_a$ (Ia)

= 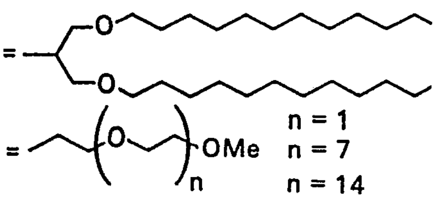 $1_b$ (Ib)

n = 1 $1_c$ (Ic)

n = 7 $1_d$ (Id)

n = 14 $1_e$ (Ie)

= 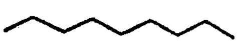 $1_f$ (If)

10. Composition according to one of claims 1 to 9, characterised in that the metal is selected from amongst the group of lanthanides and yttrium, uranium, thorium, tin and lead.

11. Composition according to claim 10, characterised in that the metal selected is lutecium.

12. The application of the compositions according to one of claims 1 to 11 to electronics or opto-electronics.

13. The application according to claim 12 to the production of electronically controlled video screens.

14. Process for the preparation of compositions according to one of claims 1 to 11, characterised in that a composition of the formula: $(R')_n(R'')_m$ Por is caused to react in di-anion form with a metallic salt of the metal Me.

15. Process according to claim 14, characterised in that the di-anion of the composition $(R')_n(R'')_m$ Por is obtained by the action of an alkali metal alcoholate.

1/6

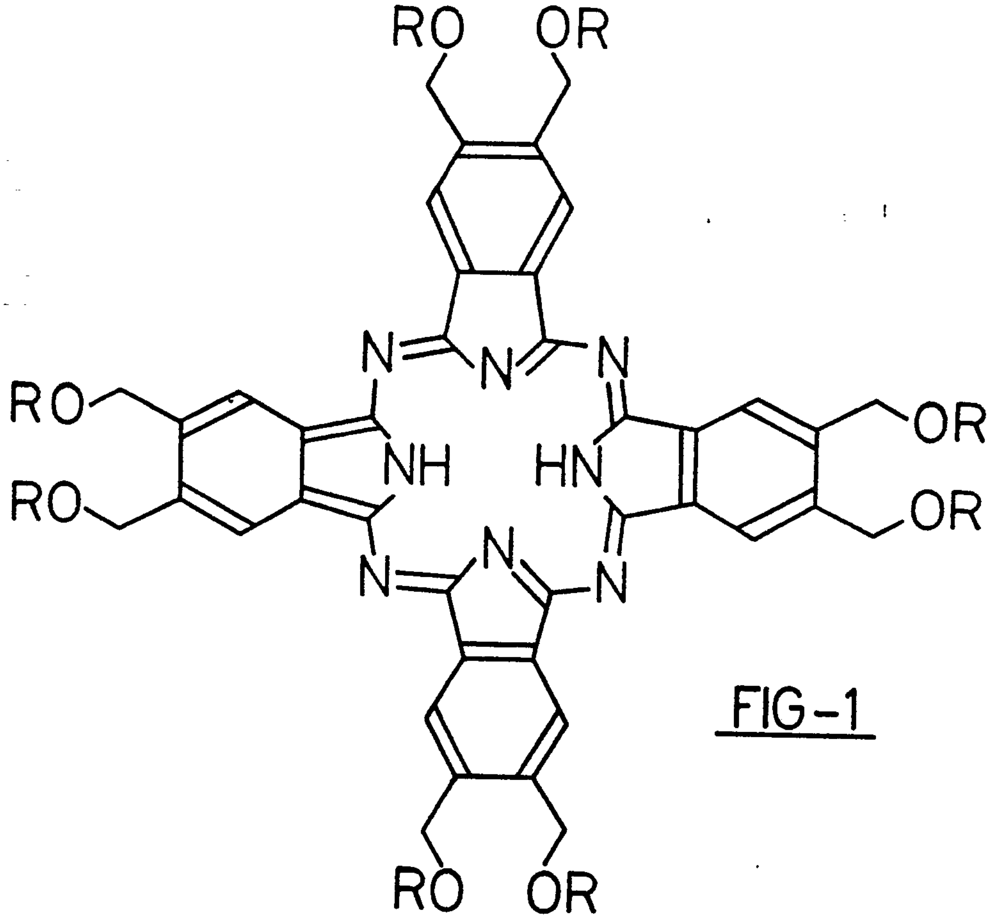

FIG-1

R = 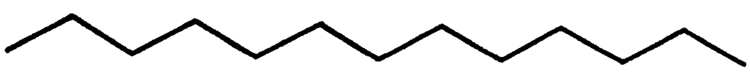 $1_a$

= 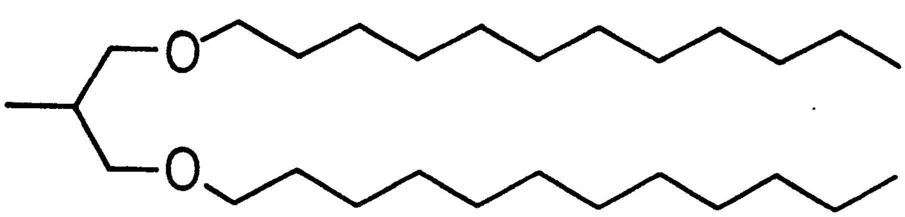 $1_b$

= 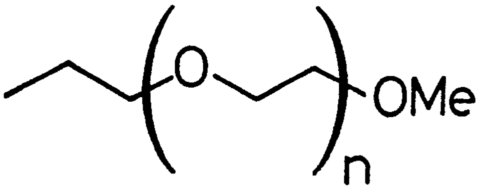

n= 1 $1_c$
n= 7 $1_d$
n= 14 $1_e$

= 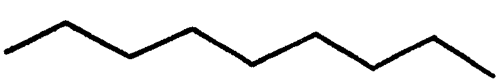 $1_f$

2/6

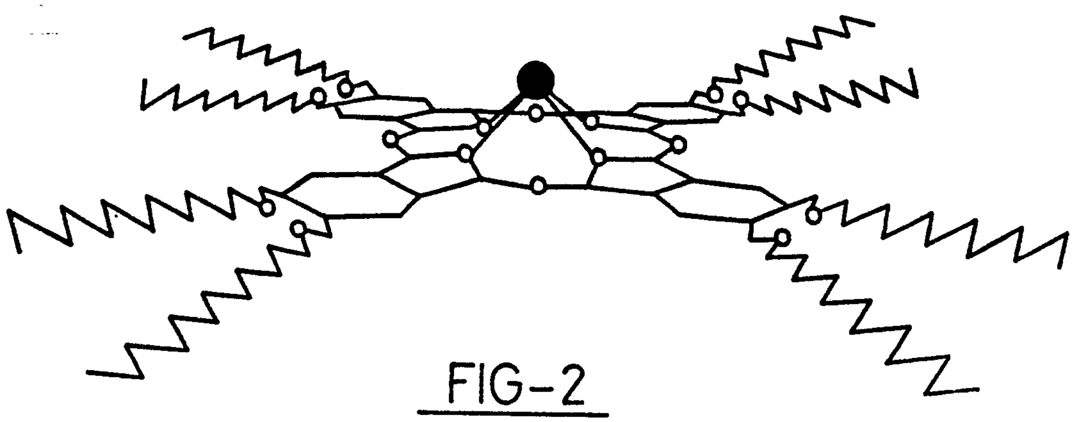

FIG-2

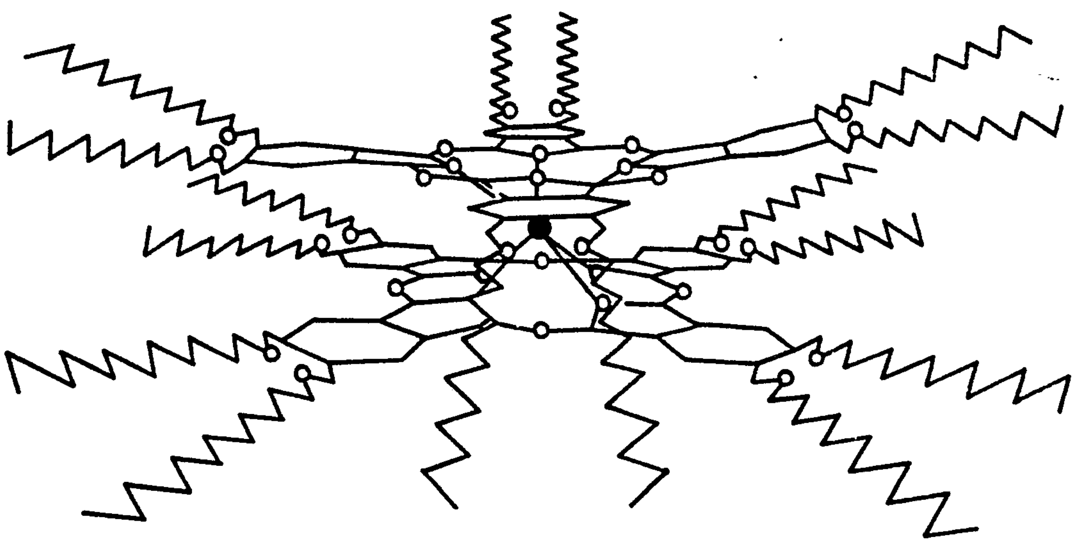

FIG-3

3/6

FIG-4

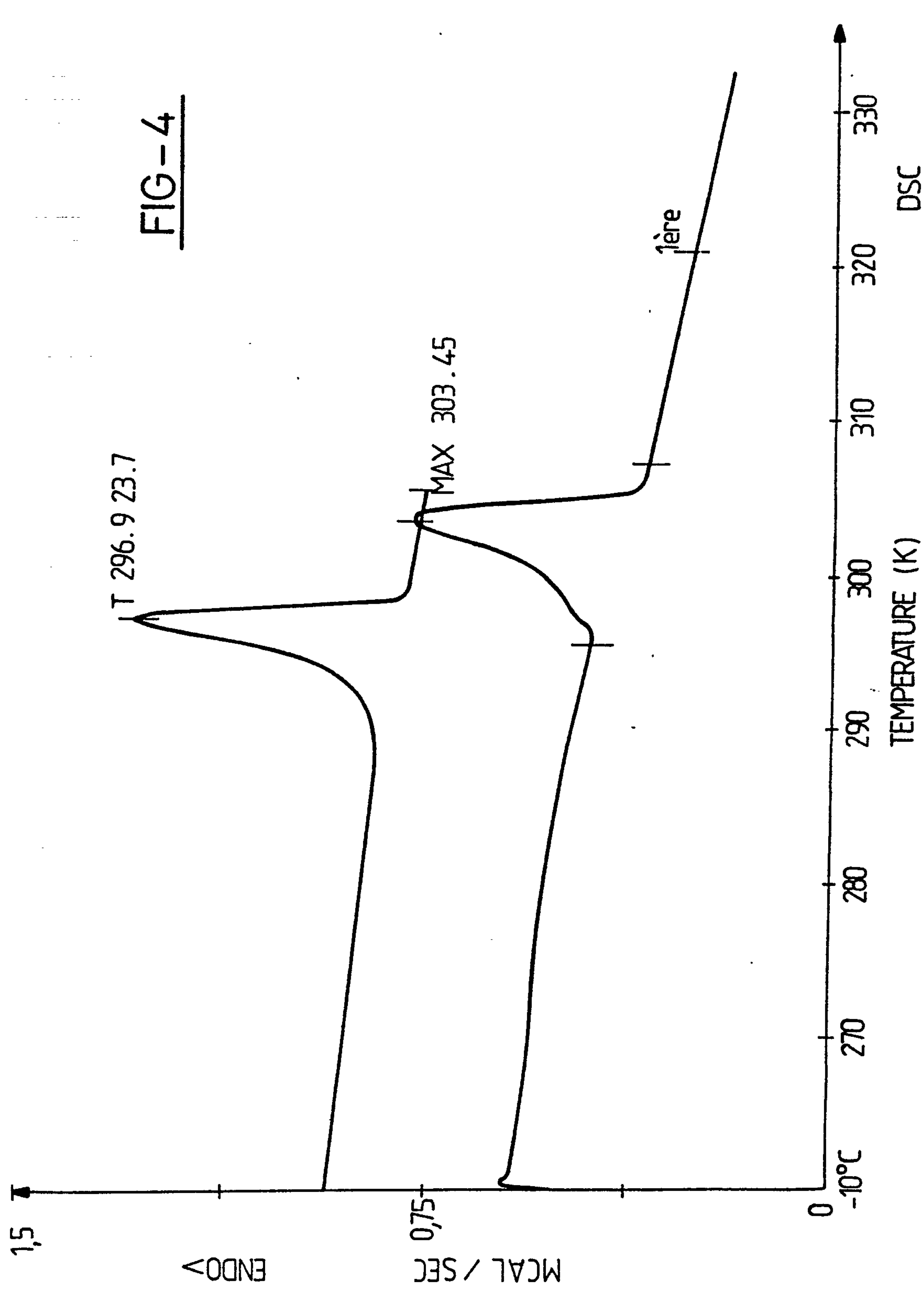
T 296.9 23.7
MAX 303.45
1ère
DSC
TEMPERATURE (K)
270
280
290
300
310
320
330
-10°C
0
0,75
1,5
MCAL / SEC
ENDO>

4 / 6

FIG–5

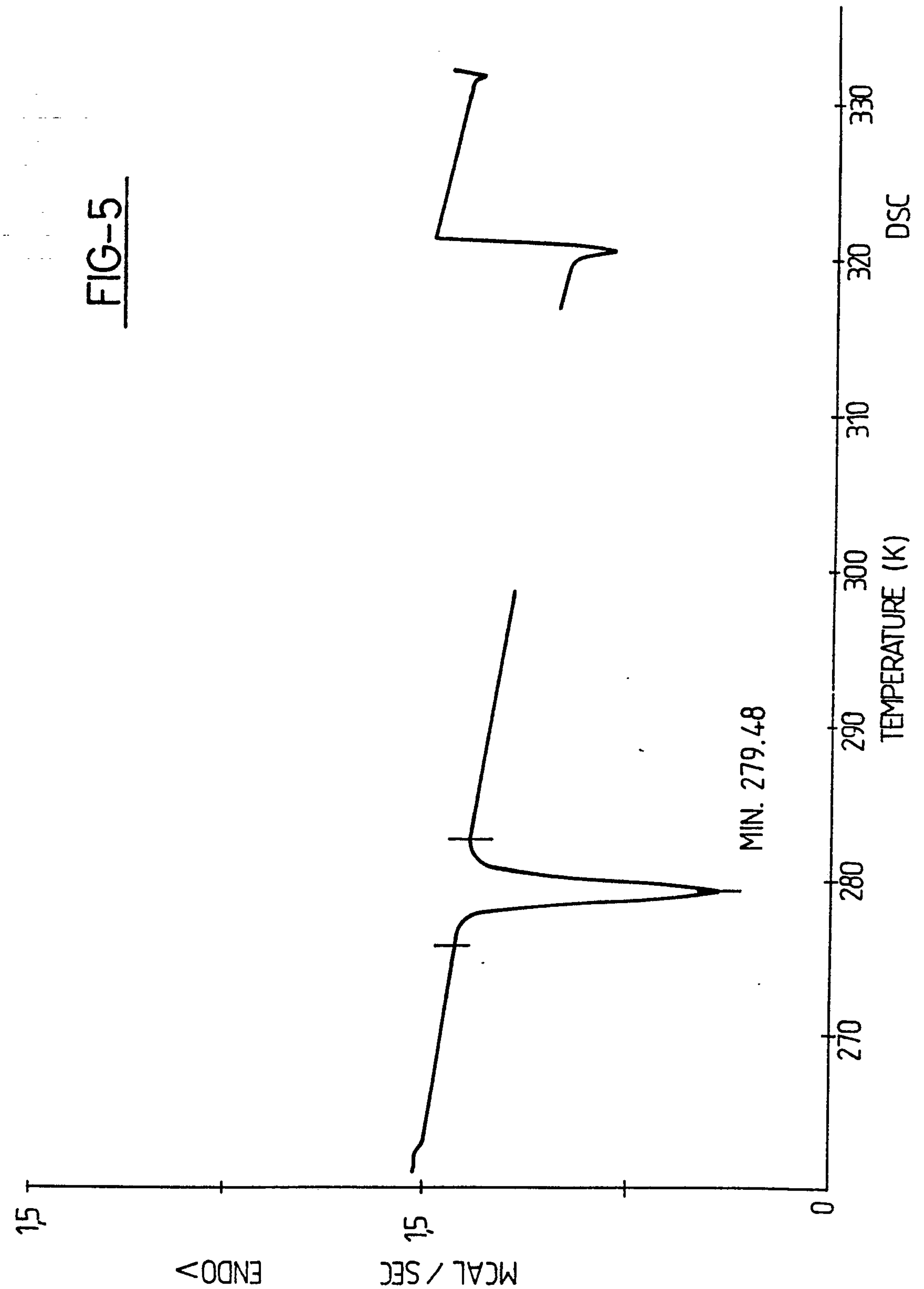
MIN. 279.48
270
280
290
300
310
320
330
TEMPERATURE (K)
DSC
0
1,5
1,5
MCAL / SEC
ENDO>

5/6

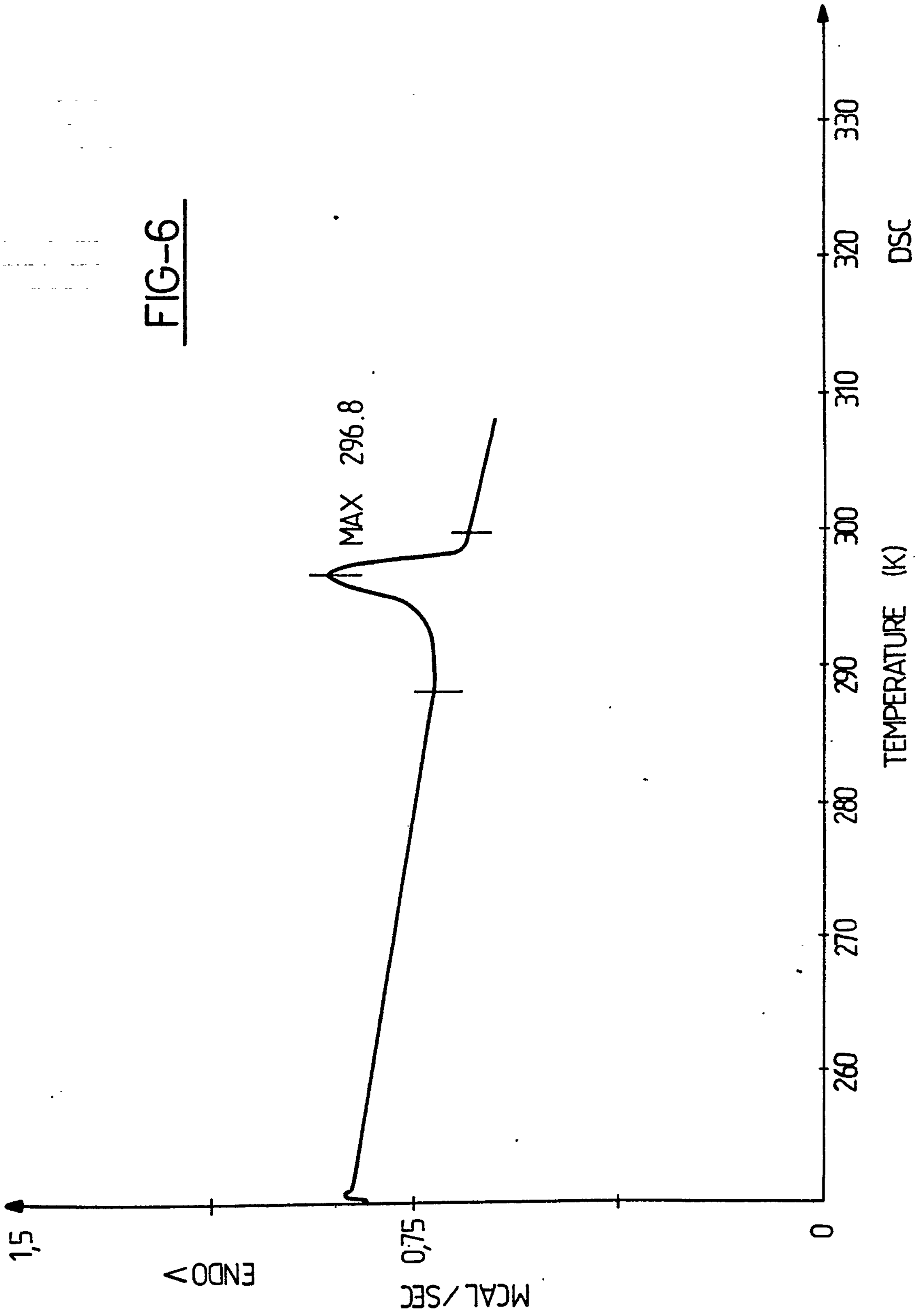
MAX 296.8
DSC
TEMPERATURE (K)
260
270
280
290
300
310
320
330
0
0,75
1,5
ENDO >
MCAL/SEC

FIG-6

6/6

FIG–7